# EUROPÄISCHE PATENTSCHRIFT

(11) **EP 2 598 464 B1**
(45) Veröffentlichungstag und Bekanntmachung des Hinweises auf die Patenterteilung: **22.01.2014**
(21) Anmeldenummer: 11738676.3
(22) Anmeldetag: 12.07.2011
(51) Int. Cl.: C10G 2/00, C07C 1/04, C10L 3/08

(54) **VERFAHREN ZUR HERSTELLUNG EINES METHANHALTIGEN GASES AUS SYNTHESEGAS SOWIE METHANGEWINNUNGSANLAGE ZUR DURCHFÜHRUNG DES VERFAHRENS**
PROCESS FOR PRODUCING A METHANE-CONTAINING GAS FROM SYNTHESIS GAS AND METHANE PRODUCTION PLANT FOR CARRYING OUT THE PROCESS
PROCÉDÉ POUR LA PRÉPARATION D'UN GAZ CONTENANT DU MÉTHANE À PARTIR D'UN GAZ DE SYNTHÈSE AINSI QU'INSTALLATION DE PRODUCTION DE MÉTHANE POUR LA RÉALISATION DU PROCÉDÉ

(30) Priorität: 28.07.2010 DE 102010032528
(43) Veröffentlichungstag der Anmeldung: 05.06.2013
(73) Patentinhaber: ThyssenKrupp Uhde GmbH, 44141 Dortmund (DE)
(72) Erfinder: MENZEL, Johannes, 45731 Waltrop (DE); THIELERT, Holger, 44379 Dortmund (DE)
(74) Vertreter: Lorenz, Bernd Ingo Thaddeus
(86) Internationale Anmeldenummer: PCT/EP2011/061893
(87) Internationale Veröffentlichungsnummer: WO 2012/013493

(56) Entgegenhaltungen:
- FR-A1- 2 290 410
- GB-A- 2 231 040
- GIERLICH H H ET AL: "Methanation of synthesis gas", 1981 INTERNATIONAL GAS RESEARCH CONFERENCE : PROCEEDINGS OF THE 1981 INTERNATIONAL GAS RESEARCH CONFERENCE, GOVERNMENT INST, ROCKVILLE, MD., USA, Bd. 2, 1. Januar 1982 (1982-01-01), Seiten 315-326, XP008143157, ISBN: 0-86587-094-2

## Beschreibung

Die Erfindung betrifft ein Verfahren zur Herstellung eines methanhaltigen Gases aus Synthesegas,
wobei ein Kohlenmonoxid und Wasserstoff enthaltendes Synthesegas zur Methanisierung einem Reaktorsystem mit einem Katalysatormaterial zugeführt wird,
wobei der das Reaktorsystem verlassende Prozessgasstrom in einen Produktgasstrom und einen Recyclegasstrom aufgeteilt wird,
wobei der Recyclegasstrom durch einen Ejektor gefördert und zur Kühlung in das Reaktorsystem (1) geleitet wird.

Mit dem Verfahren können beispielsweise Feststoffe wie Kohle, Biomasse in Form von Holz und Stroh sowie verschiedene flüssige kohlenstoffhaltige Edukte in synthetisches Erdgas umgewandelt werden, welches in ein Erdgastransportnetz eingespeist werden kann.

Die Umsetzung von Kohlenmonoxid und Wasserstoff zu Methan erfolgt einerseits gemäß der Gleichung

CO+3H₂→CH₄+H₂O.

Des Weiteren ist die folgende Gleichgewichtsreaktion

CO+H₂O ⇄ CO₂+H₂ zu berücksichtigen, so dass zusätzlich eine Erzeugung von Methan gemäß der Reaktion

CO₂+4H₂→CH₄+2H₂O

erfolgt. Die Methanisierung an einem Katalysator erfolgt insgesamt stark exotherm. Dabei ergibt sich der Nachteil, dass aufgrund der Reaktionsgleichgewichte die Methanausbeute mit steigender Temperatur abnimmt. Um das Reaktorsystem zu kühlen ist deshalb bekannt, den Prozessgasstrom, welcher das Reaktorsystem verlässt, in einen Produktgasstrom und einen Recyclegasstrom aufzuteilen, wobei der zuvor abgekühlte Recyclegasstrom wieder dem Einlass des Reaktorsystems zugeführt wird. Bei der Rückführung des Recyclegases müssen die aufgetretenen Druckverluste ausgeglichen werden. Wenn zu diesem Zweck ein Kompressor eingesetzt wird, ergibt sich das Problem, dass dieser nur mit einem erheblichen Aufwand für erhöhte Temperaturen von etwa 300°C ausgelegt werden kann, weshalb der das Reaktorsystem verlassende Prozessgasstrom üblicherweise weit heruntergekühlt wird, wobei auch eine Kondensation des in dem Prozessgasstrom enthaltenen Wassers erfolgt.

Aus der GB 1 516 319 ist bekannt, zur Förderung des Recyclegasstroms und zum Ausgleich der Druckverluste eine Strahlpumpe, die auch als Ejektor bezeichnet wird, einzusetzen. Der Ejektor ist einfach aufgebaut und kann ohne weiteres bei erhöhten Temperaturen von beispielsweise 300°C betrieben werden. Als Treibmedium des Ejektors ist das dem Reaktorsystem zugeführte Synthesegas oder Wasserdampf vorgesehen. Bei dem Einsatz von Synthesegas als Treibmedium erleidet dieses in dem Ejektor einen Druckverlust, so dass die Methanisierung in dem Reaktorsystem bei einem erniedrigten Druck erfolgt. Aufgrund der Gleichgewichte der Methanisierungsreaktionen ergibt sich durch eine Verringerung des Drucks auch eine geringere Methanausbeute, so dass sich die Effizienz des Verfahrens verringert. Die Verwendung von Wasserdampf als Treibmedium ist nachteilig, weil dadurch der Alterungsprozess üblicher Katalysatormaterialien beschleunigt wird.

Aus FR 2 290 410 A1 und Gierlich H. H. et al: "Methanation of synthesis gas", 1981 International Gas Research Conference: Proceedings of the 1981 International Gas Research Conference, Government Inst. Rockville, MD / USA, Bd. 2, 1. Januar 1982 (1982-01-01), Seiten 315 bis 326) ist der Einsatz eines Ejektors bei einem Verfahren zur Herstellung eines methanhaltigen Gases aus Synthesegas bekannt, wobei ein Kohlenmonoxid und Wasserstoff enthaltendes Synthesegas zur Methanisierung einem Reaktor mit einem Katalysatormaterial zugeführt wird. Dem Ejektor wird dabei Synthesegas oder Hochdruckdampf als Treibmedium zugeführt.

Vor diesem Hintergrund liegt der Erfindung die Aufgabe zugrunde, ein Verfahren zur Herstellung eines methanhaltigen Gases aus Synthesegas anzugeben, welches mit einer erhöhten Effizienz und geringeren Kosten durchgeführt werden kann.

Ausgehend von einem Verfahren mit den Eingangs beschriebenen Merkmalen wird diese Aufgabe erfindungsgemäß dadurch gelöst, dass der Produktgasstrom auf einem Druck komprimiert wird, der größer ist als der Druck des dem Reaktorsystem zugeführten Synthesegases, wobei entweder das so komprimierte Produktgas oder ein Nutzgas aus einem Nutzgasleitungssystem dem Ejektor als Treibmedium zugeführt wird.

Um das Produktgas einer weiteren Nutzung zuzuführen, ist eine Komprimierung vorgesehen, wobei gemäß einer ersten Ausgestaltung der Erfindung ein Anteil dieses komprimierten Produktgases als Treibmedium des Ejektors vorgesehen ist. Die bei dem Stand der Technik auftretenden Nachteile, nämlich eine Druckreduzierung des Synthesegases oder eine beschleunigte Alterung des Katalysatormaterials können damit vermieden werden.

Der Anteil des Produktgases, der nicht als Treibmedium verwendet wird, kann nach der Druckbeaufschlagung beispielsweise in einem Drucktank gelagert oder einem Nutzgasleitungssystem zugeführt werden. So kann im Rahmen der Erfindung insbesondere synthetisches Erdgas erzeugt werden, welches nach der zusätzlichen Komprimierung in ein Erdgastransportnetz eingespeist wird. Üblicherweise bewegt sich der Druck in einem solchen Nutzgasleitungssystem zwischen 60 und 80 bar, während das zur Methanisierung in das Reaktorsystem eingeleitete Synthesegas in der Regel mit einem Druck zwischen 30 und 50 bar vorliegt. Aufgrund der erheblichen Druckdifferenz wird dann nur eine verhältnismäßig kleine Menge an Treibmedium benötigt, um die Druckverluste des Recyclegasstroms ausgleichen zu können.

Wenn der Produktgasstrom nach der zusätzlichen Komprimierung in ein Nutzgasleitungssystem eingeleitet wird, besteht auch die Möglichkeit anstelle eines Anteil des Produktgasstromes Nutzgas aus dem Nutzgasleitungssystem als Treibmedium dem Ejektor zuzuführen. Insbesondere wenn das Nutzgasleitungssystem ein Erdgastransportnetz ist, ist eine Feinentschwefelung des dem Ejektor als Treibmedium zugeführten Nutzgases zweckmäßig um das Katalysatormaterial vor einer Beschädigung zu schützen. Wenn als Treibmedium für den Ejektor Nutzgas aus dem Nutzgasleitungssystem verwendet wird, ergibt sich als zusätzlicher Vorteil, dass der Ejektor auch für das Anfahren der Anlage benutzt werden kann.

Gegenstand der Erfindung ist auch eine Methangewinnungsanlage zur Durchführung des zuvor beschriebenen Verfahrens. Die Methangewinnungsanlage umfasst ein Katalysatormaterial enthaltendes Reaktorsystem zur Methanisierung, an dessen Einlass eine Zuleitung für Synthesegas angeschlossen ist, wobei an der Auslassseite des Reaktorsystems ein Leitungssystem anschließt. In einer Rückführleitung ist ein Ejektor vorgesehen, wobei eine Saugseite des Ejektors an das Leitungssystem und eine Druckseite des Ejektors an den Einlass des Reaktorsystems angeschlossen ist. Um das Recyclegas auf eine geeignete Temperatur abzukühlen, ist zumindest ein Kühler vorgesehen, der in der Rückführleitung oder vorzugsweise zwischen dem Reaktorsystem und dem Ejektor in dem Leitungssystem angeordnet ist. Insbesondere kann vorgesehen sein, den gesamten das Reaktorsystem verlassenden Prozessgasstrom durch einen zur Dampferzeugung vorgesehenen Kühler zu leiten.

Erfindungsgemäß umfasst das Leitungssystem in Strömungsrichtung gesehen hinter der Abzweigung der Rückführleitung einen Kompressor, wobei eine in einen Treibmitteleingang des Ejektors mündende Treibmittelleitung in Strömungsrichtung gesehen hinter dem Kompressor an das Leitungssystem oder an ein mit dem Leitungssystem verbundenen Nutzgasleitungssystem für methanhaltiges Gas, insbesondere ein Erdgastransportnetz, angeschlossen ist.

Da in einem einzelnen Reaktor aufgrund der chemischen Gleichgewichte nur ein begrenzter Methanumsatz von beispielsweise 20 % erreicht wird, weist das Reaktorsystem zweckmäßigerweise mehrere hintereinander geschaltete Reaktorstufen auf, die jeweils Katalysatormaterial aufweisen. Im Rahmen der Erfindung liegt es auch, das Produktgas zwischen einzelnen Reaktorstufen herunterzukühlen. Je nach Anwendungsfall können noch weitere Aufbereitungsschritte des Synthesegases wie beispielsweise die Gastrocknung und die Entfernung von CO₂ vorgesehen sein, wobei diese Aufbereitungsschritte innerhalb des Reaktorsystems oder getrennt von dem Reaktorsystem realisiert werden können.

Die Erfindung wird im Folgenden anhand einer lediglich ein Ausführungsbeispiel darstellenden Zeichnung erläutert. Es zeigen:
- Fig. 1: eine Methangewinnungsanlage zur Herstellung eines methanhaltigen Gases aus Synthesegas,
- Fig. 2: eine Variante der in Fig. 1 dargestellten Methangewinnungsanlage.

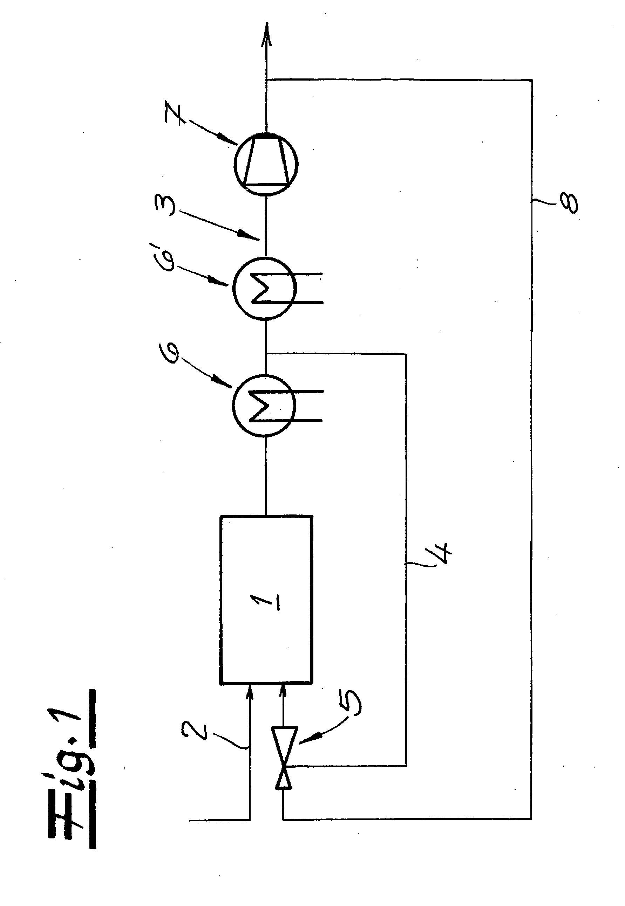

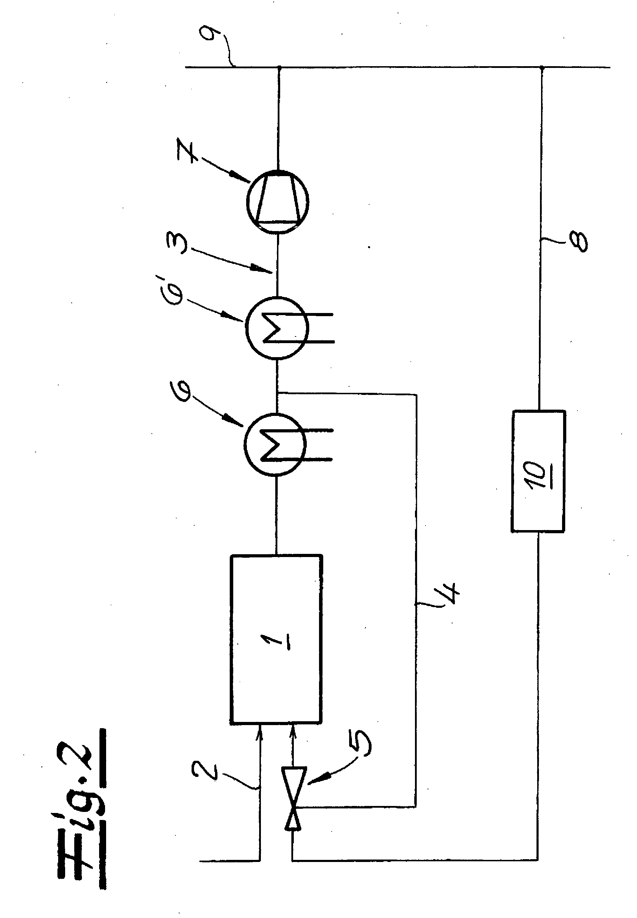

Die Fig. 1 und 2 zeigen eine Methangewinnungsanlage mit einem Katalysatormaterial enthaltenden Reaktorsystem 1 zur Methanisierung eines Kohlenmonoxid und Wasserstoff enthaltenden Synthesegases. An den Einlass des Reaktorsystems 1 ist eine Zuleitung 2 für das Synthesegas angeschlossen, wobei an die Auslassseite des Reaktorsystems 1 ein Leitungssystem 3 anschließt. Der das Reaktorsystem 1 verlassende Prozessgasstrom wird in einen Produktgasstrom und einen Recyclegasstrom aufgeteilt, wobei der Recyclegasstrom durch eine Rückführleitung 4 mittels eines Ejektors 5 zurück zu dem Einlass des Reaktorsystems 1 geführt wird. Der Ejektor 5 ist dazu mit einer Saugseite an das Leitungssystem 3 und mit einer Druckseite an den Einlass des Reaktorsystems 1 angeschlossen. Zwischen dem Reaktorsystem 1 und dem Ejektor 5 ist ein Kühler 6 in dem Leitungssystem 3 angeordnet.

Das Leitungssystem 3 umfasst in Strömungsrichtung gesehen hinter der Abzweigung der Rückführleitung 4 einen Kompressor 7, welcher den Produktgasstrom weiter komprimiert. Zwischen der Abzweigung der Rückführleitung 4 und dem Kompressor 7 ist mindestens ein weiterer Kühler 6' angeordnet. Dieser dient dazu die Temperatur des Produktgasstroms soweit abzusenken, dass eine spezielle Auslegung des Kompressors 7 für hohe Temperaturen nicht notwendig ist.

Gemäß der Fig. 1 wird ein Anteil des komprimierten Produktgasstroms hinter dem Kompressor 7 ausgeschleust und über eine Treibmittelleitung 8 zu einem Teibmitteleingang des Ejektors 5 geleitet. Während das Synthesegas in der Zuleitung 2 mit einem Druck von typischerweise zwischen 30 und 50 bar in das Reaktorsystem 1 eingeleitet wird, beträgt der Druck des Produktgases hinter dem Kompressor vorzugsweise zwischen 60 und 80 bar. Aufgrund des deutlich höheren Druckes ist ein kleiner Anteil des Produktgasstromes ausreichend, um den Recyclegasstrom zu fördern und die entsprechenden Druckverluste ausgleichen zu können. Gemäß der Fig. 1 kann der Anteil des nicht als Treibmedium genutzten Produktgases zur weiteren Nutzung ohne Einschränkung einem Druckspeicher oder einem Nutzgasleitungssystem zugeführt werden.

Fig. 2 zeigt eine Ausgestaltung, bei der im Unterschied zu der Fig. 1 das gesamte Produktgas nach der Komprimierung durch den Kompressor 7 einem Nutzgasleitungssystem 9, beispielsweise einem Erdgastransportnetz, zugeführt wird. Die Treibmittelleitung 8 ist dabei an das Nutzgasleitungssystem 9 angeschlossen. Mit der dargestellten Methangewinnungsanlage kann beispielsweise synthetisches Erdgas (SNG) in ein Erdgastransportnetz eingespeist werden. Es ergibt sich der Vorteil, dass die Treibmittelleitung 8 auch zum Anfahren der Methangewinnungsanlage genutzt werden kann. Um das Reaktorsystem 1 vor Schwefelkomponenten zu schützen, die in dem Nutzgas enthalten sein können, ist gemäß der Fig. 2 in der Treibmittelleitung 8 eine Vorrichtung 10 zur Feinentschwefelung vorgesehen.

## Patentansprüche

1. Verfahren zur Herstellung eines methanhaltigen Gases aus Synthesegas,
wobei ein Kohlenmonoxid und Wasserstoff enthaltendes Synthesegas zur Methanisierung einem Reaktorsystem (1) mit einem Katalysatormaterial zugeführt wird,
wobei der das Reaktorsystem (1) verlassende Prozessgasstrom in einen Produktgasstrom und einen Recyclegasstrom aufgeteilt wird,
wobei der Recyclegasstrom durch einen Ejektor (5) gefördert und zur Kühlung in das Reaktorsystem (1) geleitet wird,
**dadurch gekennzeichnet, dass** der Produktgasstrom auf einen Druck komprimiert wird, der größer ist als der Druck des dem Reaktorsystem (1) zugeführten Synthesegases und dass komprimiertes Produktgas dem Ejektor (5) als Treibmedium zugeführt wird.

2. Verfahren zur Herstellung eines methanhaltigen Gases aus Synthesegas,
wobei ein Kohlenmonoxid und Wasserstoff enthaltendes Synthesegas zur Methanisierung einem Reaktorsystem (1) mit einem Katalysatormaterial zugeführt wird,
wobei der das Reaktorsystem (1) verlassende Prozessgasstrom in einen Produktgasstrom und einen Recyclegasstrom aufgeteilt wird,
wobei der Recyclegasstrom durch einen Ejektor (5) gefördert und zur Kühlung in das Reaktorsystem (1) geleitet wird,
**dadurch gekennzeichnet, dass** der Produktgasstrom auf einen Druck komprimiert wird, der größer ist als der Druck des dem Reaktorsystem (1) zugeführten Synthesegases, dass der Produktgasstrom in ein Nutzgasleitungssystem (9) eingeleitet wird und dass dem Ejektor (5) Nutzgas aus dem Nutzgasleitungssystem (9) als Treibmedium zugeführt wird.

3. Verfahren nach Anspruch 2, **dadurch gekennzeichnet, dass** das dem Ejektor (5) als Treibmedium zugeführte Nutzgas einer Feinentschwefelung unterzogen wird.

4. Verfahren nach Anspruch 2 oder 3, **dadurch gekennzeichnet, dass** das Nutzgasleitungssystem (9) ein Erdgastransportnetz ist.

5. Verfahren nach einem der Ansprüche 1 bis 4, **dadurch gekennzeichnet, dass** das Synthesegas mit einem Druck zwischen 30 und 50 bar dem Reaktorsystem (1) zugeführt wird.

6. Verfahren nach einem der Ansprüche 1 bis 5, **dadurch gekennzeichnet, dass** der Produktgasstrom auf einen Druck zwischen 60 und 80 bar komprimiert wird.

7. Methangewinnungsanlage zur Durchführung des Verfahrens nach einem der Ansprüche 1 bis 6 mit
einem ein Katalysatormaterial enthaltenden Reaktorsystem (1) zur Methanisierung, an dessen Einlass eine Zuleitung (2) für Synthesegas angeschlossen ist,
einem an die Auslassseite des Reaktorsystems (1) angeschlossenen Leitungssystem (3),
einem Ejektor (5) in einer Rückführleitung (4), wobei eine Saugseite des Ejektors (5) an das Leitungssystem (3) und eine Druckseite des Ejektors (5) an den Einlass des Reaktorsystems (1) angeschlossen ist,
zumindest einem Kühler (6), der in der Rückführleitung (4) oder zwischen dem Reaktorsystem (1) und dem Ejektor (5) in dem Leitungssystem (3) angeordnet ist,
**dadurch gekennzeichnet, dass** das Leitungssystem (3) in Strömungsrichtung gesehen hinter der Abzweigung der Rückführleitung (4) einen Kompressor (7) umfasst, wobei eine in einen Treibmitteleingang des Ejektors (5) mündende Treibmittelleitung (8) in Strömungsrichtung gesehen hinter dem Kompressor (7) an das Leitungssystem (3) oder an ein mit dem Leitungssystem (3) verbundenen Nutzgasleitungssystem (9) für methanhaltiges Gas angeschlossen ist.

## Claims

1. A method of making a methane-containing gas from synthesis gas,
wherein a synthesis gas containing carbon monoxide and hydrogen is supplied to a reactor system (1) holding a catalytic material to effect methanation,
wherein the process gas stream leaving the reactor system (1) is divided into a product-gas stream and a recycle-gas stream,
wherein the recycle-gas stream is moved through an ejector (5) and conducted into the reactor system (1) for cooling,
**characterized in that** the product-gas stream is compressed to a pressure that is greater than the pressure of the synthesis gas supplied to the reactor system (1) and the compressed product-gas stream is supplied to the ejector (5) as the motive medium.

2. The method of making a methane-containing gas from synthesis gas,
wherein a synthesis gas containing carbon monoxide and hydrogen is supplied to a reactor system (1) holding a catalyst material to effect methanation,
wherein the process gas stream leaving the reactor system (1) is divided into a product-gas stream and a recycle-gas stream,
wherein the recycle-gas stream is moved through an ejector (5) and conducted into the reactor system (1) for cooling, **characterized in that** the product-gas stream is compressed to a pressure that is greater than the pressure of the synthesis gas supplied to the reactor system (1), that the product-gas stream is introduced into a useful-gas conduit system (9) and that useful gas from the useful-gas conduit system (9) is supplied to the ejector (5) as the motive medium.

3. The method according to claim 2, **characterized in that** the useful gas supplied as the motive medium to the ejector (5) is made to undergo fine desulfurization.

4. The method according to claim 2 or 3, **characterized in that** the useful-gas conduit system (9) is a natural-gas transmission network.

5. The method according to one of the claims 1 to 4, **characterized in that** the synthesis gas is supplied to the reactor system (1) at a pressure of between 30 and 50 bar.

6. The method according to one of the claims 1 to 5, **characterized in that** the product-gas stream is compressed to a pressure of between 60 and 80 bar.

7. A methane plant for carrying out the method according to one of the claims 1 to 6 having
a reactor system (1) holding a catalyst material to effect methanation and having an inlet to which a supply line (2) for synthesis gas is connected,
a conduit system (3) connected to an outlet side of the reactor system (1),
an ejector (5) in a recirculation line (4), wherein a suction side of the ejector (5) is connected to the conduit system (3) and a pressure side of the ejector (5) connected to the inlet of the reactor system (1),
at least one cooler (6) provided in the recirculation line (4) or in the conduit system (3) between the reactor system (1) and the ejector (5),
**characterized in that** the conduit system (3) downstream in a flow direction of the branching point of the recirculation line (4) comprises a compressor (7), wherein a motive medium line (8) that discharges into a motive medium inlet of the ejector (5) is connected to the conduit system (3) downstream of the compressor (7) in the flow direction or is connected to a useful-gas conduit system (9) for methane-containing gas that is connected to the conduit system (3).

## Revendications

1. Procédé permettant de fabriquer, à partir de gaz de synthèse, un gaz contenant du méthane,
un gaz de synthèse, qui contient du monoxyde de carbone et de l'hydrogène, étant acheminé vers un système de réacteur (1) comportant une matière catalytique, pour y être transformé en méthane,
le flux de gaz ainsi traité étant divisé, lorsqu'il qui sort du système de réacteur (1), en un flux de gaz de produit et un flux de gaz à recycler,
le flux de gaz à recycler étant transporté à travers un éjecteur (5) et introduit dans le système de réacteur (1) pour le refroidir,
**caractérisé en ce que** le flux de gaz de produit est comprimé jusqu'à atteindre une pression supérieure à la pression du gaz de synthèse acheminé vers le système de réacteur (1) et que du gaz de produit comprimé est acheminé vers l'éjecteur (5) en tant que milieu d'entraînement.

2. Procédé permettant de fabriquer, à partir de gaz de synthèse, un gaz contenant du méthane,
un gaz de synthèse, qui contient du monoxyde de carbone et de l'hydrogène, étant acheminé vers un système de réacteur (1) comportant une matière catalytique, pour y être transformé en méthane,
le flux de gaz ainsi traité étant divisé, lorsqu'il qui sort du système de réacteur (1), en un flux de gaz de produit et un flux de gaz à recycler,
le flux de gaz à recycler étant transporté à travers un éjecteur (5) et introduit dans le système de réacteur (1) pour le refroidir,
**caractérisé en ce que** le flux de gaz de produit est comprimé jusqu'à atteindre une pression supérieure à la pression du gaz de synthèse acheminé vers le système de réacteur (1), que le flux de gaz de produit est introduit dans un système de conduites de gaz utile (9), et que du gaz utile est prélevé dans le système de conduites de gaz utile (9) pour l'acheminer vers l'éjecteur (5) en tant que milieu d'entraînement.

3. Procédé selon la revendication 2, **caractérisé en ce que** le gaz utile, qui est acheminé vers l'éjecteur (5) en tant que milieu d'entraînement, est soumis à une désulfuration poussée.

4. Procédé selon la revendication 2 ou 3, **caractérisé en ce que** le système de conduites de gaz utile (9) est un réseau de transport de gaz naturel.

5. Procédé selon l'une des revendications 1 à 4, **caractérisé en ce que** le gaz de synthèse est acheminé vers le système de réacteur (1) sous une pression entre 30 et 50 bar.

6. Procédé selon l'une des revendications 1 à 5, **caractérisé en ce que** le flux de gaz de produit est comprimé jusqu'à atteindre une pression entre 60 et 80 bar.

7. Installation de production de méthane destinée à la mise en oeuvre du procédé selon l'une des revendications 1 à 6, comportant
un système de réacteur (1) qui contient une matière catalytique et est destiné à la transformation en méthane, une ligne d'alimentation (2) en gaz de synthèse étant raccordée à son entrée,
un système de conduites (3) qui est raccordé au côté sortie du système de réacteur (1),
un éjecteur (5) au sein d'une conduite de recyclage (4), un côté aspiration de l'éjecteur (5) étant raccordé au système de conduites (3) et un côté pression de l'éjecteur (5) étant raccordé à l'entrée du système de réacteur (1),
au moins un réfrigérant (6) qui est disposé dans la conduite de recyclage (4) ou bien, au sein du système de conduites (3), entre le système de réacteur (1) et l'éjecteur (5),
**caractérisée en ce que** le système de conduites (3) comprend un compresseur (7) situé, dans le sens de l'écoulement, en aval du site de branchement de la conduite de recyclage (4) alors qu'une conduite de milieu d'entraînement (8), laquelle débouche sur une entrée de milieu d'entraînement de l'éjecteur (5), est raccordée, en aval du compresseur (7) en suivant le sens de l'écoulement, au système de conduites (3) ou à un système de conduites de gaz utile (9) qui est relié au système de conduites (3) et qui est destiné à un gaz contenant du méthane.
